# EUROPEAN PATENT SPECIFICATION

(11) **EP 0 977 562 B1**
(45) Date of publication and mention of the grant of the patent: **04.01.2006**
(21) Application number: 99909941.9
(22) Date of filing: 10.03.1999
(51) Int. Cl.: A61K 31/335, A61K 47/22

(54) **METHODS AND COMPOSITIONS FOR DELIVERY OF TAXANES**
VERFAHREN UND ZUSAMMENSETZUNGEN ZUR VERABREICHUNG VON TAXANEN
METHODES ET COMPOSITIONS D'ADMINISTRATION DE TAXANES

(30) Priority: 10.03.1998 US 77459 P
(43) Date of publication of application: 09.02.2000
(73) Proprietor: NAPRO BIOTHERAPEUTICS, INC., Boulder, CO 80301 (US)
(72) Inventor: McCHESNEY-HARRIS, Lisa, L., Vernon Hills, IL 60061-1317 (US)
(74) Representative: Lawrence, Malcolm Graham
(86) International application number: PCT/US1999/005151
(87) International publication number: WO 1999/045918

(56) References cited:
- WO-A-95/11039
- WO-A-98/30205
- CHANG T ET AL: "THE EFFECT OF WATER-SOLUBLE VITAMIN E (TPGS) ON ORAL CYCLOSPORINE PHARMACOKINETICS IN HEALTHY VOLUNTEERS" CLINICAL PHARMACOLOGY & THERAPEUTICS, vol. 57, no. 1, 1 January 1995, XP000196224
- ADAMS M.W.: "d-alpha Tocopheryl Polyethylene Glycol 1000 Succinate (Eastman Vitamin E TPGS) as an Emulsifier and Bioenhancer for Drugs and Lipophilic Compounds" CONGR. INT. TECHNOL. PHARM./6TH, vol. 4, 1992, pages 254-262, XP002011712

## Description

### Background of the Invention

Taxanes make up an important class of cytotoxic agents which have been the subject of much interest and research directed to producing new and improved cancer-fighting therapies. A particularly promising taxane, paclitaxel, is a compound extracted from the bark of a western yew, *Taxus brevifolia* and known for its antineoplastic activity. It is described, for example, in The Merck Index, Eleventh Edition 1989, monograph 9049.

In 1977, paclitaxel was chosen for development as an antineoplastic agent because of its unique mechanism of action and good cytotoxic activity against IP implanted D16 melanoma and the human X-1 mammary tumor xenograft.

Paclitaxel is believed to function as a mitotic spindle poison and as a potent inhibitor of cell replication *in vitro.* Other mitotic spindle poisons (colchicine and podophyllotoxin) inhibit microtubule assembly. Paclitaxel employs a different mechanism of action since it appears to shift the equilibrium of polymerization/depolymerization toward polymer assembly and to stabilize microtubules against depolymerization under conditions which would cause rapid desegregation of microtubules. The interference with the polymerization/depolymerization cycle in cells appears to interfere with both the replication and migration of cells.

Paclitaxel has demonstrated good response rates in treating both ovarian and breast cancer patients who were not benefitting from vinca alkaloid or cisplatin therapy. It has also shown encouraging results in patients with other types of cancer including lung, melanoma, lymphoma, head, and neck. For further information, reference may be made to the U.S. National Cancer Institute's Clinical Brochure for Taxol, revised July 1991, and papers presented at the Second National Cancer Institute Workshop on Taxol and Taxus held in Alexandria, Virginia USA on September 23-24, 1992.

Despite these studies which affirm paclitaxel's importance as a tool in the fight against cancer, the chemical structure of paclitaxel creates obstacles for its efficient pharmaceutical administration. One such obstacle is that paclitaxel is water insoluble and tends to precipitate when placed in an aqueous solution. Some formulations of paclitaxel used for injection or IV infusion have been developed primarily utilizing CREMOPHOR® EL as the drug carrier to overcome the low water solubility problems of paclitaxel. Cremophor, however, is itself somewhat toxic, causing idiosyncratic histamine release and anaphylactoid like response. Thus, the use of this carrier is not a desirable solution to the problem of developing good formulations of taxanes.

Extensive efforts have been made to circumvent these problems inherent in the administration of paclitaxel. For example, in U.S. Patent No. 4,942,184, Haugwitz *et al.* attempted to make paclitaxel more water soluble by altering its chemical structure. See also U.S. Patent No. 4,960,790. This changing of the chemical structure of paclitaxel can potentially decrease the antitumor activity of the drugs, and does not address the problem of low stability and short shelf life,

There is continuing need for taxane compositions and formulations which provide a more efficient means of administering taxanes without causing allergic reactions or other undesired side effects, and which have improved stability and longer shelf life.

### Summary of the Invention

The subject invention pertains to novel compositions for delivery of paclitaxel and other taxanes or their water insoluble derivatives. Specifically exemplified are compositions of paclitaxel solubilized in d-alpha-tocopheryl polyethylene glycol 1000 succinate and methods of making the same. The compositions of the subject invention and the methods described herein for making the compositions, provide paclitaxel compositions which have improved stability and are suitable for oral or injectable administration.

Described herein are methods of preparing taxane formulations comprising mixing Vitamin E TPGS with an organic solvent to form a carrier solution and contacting a taxane with said carrier solution, whereby said taxane is solubilized in said carrier solution and does not readily degrade. An acid may be added to the organic solvent to reduce its pH before mixing with the Vitamin E TPGS, which acts to improve stability of the taxane. U.S. Patent No. 5,733,888 teaches methods for stabilizing paclitaxel by reducing pH of the carrier solution.

The subject invention pertains to novel compositions comprising a taxane, wherein said taxane is solubilized in Vitamin E TPGS micelles. The subject invention encompasses compositions designed for oral administration or injectable administration.

In one aspect, the subject invention provides a composition comprising a taxane and Vitamin E-TPGS and at least one of:
(a) PEG 300, methoxy PEG 350 or PEG 4600;
(b) Dimethylisosorbide (DMI); and
(c) an organic solvent from 40% to 75% of the composition:

Or
A composition comprising 25 to 75% Vitamin E-TPGS, 25 to 75% ethanol, and 0.6 to 5% taxane.

In yet another aspect, the subject invention provides use of a taxane and Vitamin E-TPGS for the manufacture of a medicament for treating a taxane-responsive disease condition which medicament comprises a taxane and Vitamin E-TPGS and at least one of:
(a) PEG 300, methoxy PEG 350 or PEG 4600;
(b) Dimethylisosorbide (DMI): and
(c) an organic solvent which if present is from 40% to 75% of the composition

Or
wherein said medicament comprises 25 to 75% Vitamin E-TPGS. 25 to 75% ethanol, and 0.6 to 5% taxane.

### Detailed Disclosure of the Invention

The subject invention pertains to novel taxane compositions. Also disclosed herein are methods of making the same. Specifically exemplified herein are compositions comprising taxanes, Vitamin E TPGS, and an organic solvent.

One aspect of the subject invention is directed to compositions comprising taxanes, Vitamin E TPGS, and ethanol. A specific embodiment of the subject invention utilizes paclitaxel as the taxane component.

The compositions of the subject invention may be formulated with or without further excipients. Examples of preferred compositions include, but are not limited to, the following:
a) solutions for drinking,
b) emulsions for drinking,
c) injection solutions, and
d) solutions contained in capsules.

The modes of administration may include, but are not limited to intramuscular, subcutaneous, intravenous, parenteral, and oral administration.

In a specific aspect, the pH of the carrier composition can be reduced to further improve the stability of the taxane contained in said composition. In some embodiments, this is accomplished by the addition of an acid. In a preferred embodiment, the acid is citric acid.

In other embodiments, the compositions of the subject invention can be contained within a gelatin capsule. Thickeners known in the art can be added to these compositions in order to make them more suitable for gelatin capsule administration. An example of such a thickener includes, but is not limited to, PEG 4600.

A taxane formulation can be prepared using a method comprising mixing Vitamin E TPGS with an organic solvent to form a carrier solution, and then contacting a taxane with said carrier solution, whereby said taxane is solubilized in said carrier solution and does not readily degrade. The desired amount of Vitamin E TPGS may be warmed to approximately 40°C, and then stirred as the organic solvent is added. Organic solvents which can be used in the subject method include, but are not limited to, ethanol The contacting of taxane with the carrier solution can be accomplished by adding the taxane to the carrier solution slowly, with continued stirring, at 40°C. The resulting composition can remain a fluid even after cooling to ambient temperature. The resulting composition can be maintained as an anhydrous solution. Upon administration of this anhydrous solution, it contacts aqueous bodily fluids whereby the solution emulsifies and forms taxane-containing micelles.

The percentages of the respective components of the subject compositions will vary depending on the type of administration contemplated. Compositions most suitable for oral administration will, in preferred embodiments, have a more solid or semi-solid consistency. Accordingly, compositions for oral administration will preferably comprise about 50% to about 75% Vitamin E-TPGS. in contrast, the most preferred embodiments of compositions ultimately intended for intravenous (IV) or parenteral administration, for example, will preferably comprise lesser amounts of Vitamin B-TPGS, ideally between about 25% to about 60% Vitamin E-TPGS. Compositions ultimately intended for IV or parenteral administration can also comprise an organic solvent, in an amount of about 40% to about 75%.

The present invention may be understood more readily by reference to the following descriptions of preferred embodiments and examples of the invention. Other embodiments within the scope of the invention will be readily apparent to those of skill in the art in view of the teachings herein.

### Example 1 - Surfactant/Solvent Combinations Comprising a Taxane Component

4.040g of Vitamin E-TPGS (Eastman Chemical Co., Kingsport, TN) was chipped in and weighed into a 20 ml scintillation vial. 0.514g of dimethylisosorbide ("DMI"; ARLASOLVE® DMI, ICI Surfactants; Wilmington, DE) was added to the vial followed by heating to melt the Vitamin E-TPGS/DMI mixture. Upon liquification of the Vitamin E-TPGS/DMI mixture, 0.248g of paclitaxel was added with stirring. Unexpectedly, the taxane rapidly dispersed and the particles solubilized quickly. The solution began to clarify and dissolution progressed. The solution composition was adjusted to determine how much paclitaxel it could efficiently solubilize. Accordingly, additional amounts of Vitamin E-TPGS/DMI, and paclitaxel were added to the mixture with stirring. The majority of the bulk paclitaxel dissolved in the warmed, stirred matrix. Final concentrations were as follows: 7.065g Vitamin E-TPGS, 2.014g dimethylisosorbide, and 0.664g paclitaxel.

To test the potential for drug precipitation, a small quantity of the warmed mixture was transferred via a plastic transfer pipette to a 20 ml scintillation vial containing cold water. The drop of formulation immediately congealed (solidified) on the surface of the water and formed a clear, gelatinous-like mass. Upon agitation, the mass dissolved and a large number of bubbles were visible on the surface of the solution. However, there was no visible precipitation of any drug particles. Approximately 24 hours later, there still appeared to be many bubbles at the surface and the presence of a very thin, opaque film could be seen on the bottom of the vial. This sedimentation was possibly paclitaxel that had partitioned out of the micelles.

The remaining original Vitamin E-TPGS, DMI, and paclitaxel solution mixture was also allowed to cool to room temperature. The mixture congealed to form a semi-solid, light amber yellow mass. No apparent phase separation could be seen to indicate possible incompatibility. No crystals appeared to be present in the matrix.

A small quantity of congealed formulation was transferred to a separate 20 ml scintillation vial and 3 to 4 ml of warm water were added. The sample was agitated gently and visually monitored. The semi-solid mass quickly hydrated and became translucent with no evidence of precipitation of active components either within the gelatinous matrix or in the aqueous phase.

The experiment as described above was repeated, and the results were reproducible.

### Example 2 - Vitamin E-TPGS Formulation Optimization for Solubilizing Paclitaxel

Various concentrations of GREMOPHOR® EL/citric acid blend, DMI, Vitamin E-TPGS, and paclitaxel were mixed together to determine preferred formulations for solubilizing paclitaxel. See Table 1. Each formulation was prepared in a 20 ml scintillation vial. The vial was initially tared. CREMOPHOR® EL/citric acid blend (10g CREMOPHOR® EL, 0.020g citric acid) was weighed into each of eight (8) tared vials. The weight was recorded. Each vial was subsequently tared and the appropriate weight of dimethylisosorbide (DMI) was added. An additional five (5) vials were also prepared without containing the CREMOPHOR® EL/citric acid blend. These are identified as Samples 9-13 of Table 1. All vials were individually tared and the appropriate weight of Vitamin E-TPGS was added. At this point, all vials were placed in a 40°C incubator to liquefy the Vitamin E-TPGS within each sample. After the solutions had liquefied, two samples were removed at a time and placed on a stir/hotplate. One-half inch egg shaped stir bars were added to each scintillation vial.

Small weighing canoes were tared on the balance and paclitaxel was transferred until the desired weight was achieved. Large clumps were broken apart easily with a stainless steel spatula. The paclitaxel was slowly added to the warmed, stirring mixtures of Samples 1 and 2. Despite the presence of CREMOPHOR® EL, the bulk drug dissipated quickly and the solutions clarified relatively quickly. The last few particles took a little longer to dissolve, but eventually did, and the taxane retained its solubility even in the presence of 10% CREMOPHOR® EL. This same procedure was repeated for each of the samples.

Formulations were prepared as above wherein the DMI was substituted by methoxylated PEG 350. See Table 2.

| **Table 1.** | | | | | | | | |
|---|---|---|---|---|---|---|---|---|
| **Sample ID** | **CREMOPHOR® EL, Citric Acid Blend** | | **DMI** | | **Vitamin E-TPGS** | | **Paclitaxel** | |
| | *Desired Amount* | *Actual Amount* | *Desired Amount* | *Actual Amount* | *Desired Amount* | *Actual Amount* | *Desired Amount* | *Actual Amount* |
| 1 | 0.50 g | 0.498 | 1.00 g | 1.013 | 3.25 g | 3.269 | 0.25 g | 0.251 |
| 2 | 0.50 g | 0.498 | 1.00 g | 1.006 | 3.20 g | 3.206 | 0.30 g | 0.304 |
| 3 | 0.50 g | 0.507 | 1.00 g | 1.019 | 3.15 g | 3.158 | 0.35 g | 0.350 |
| 4 | 0.50 g | 0.499 | 1.25 g | 1.252 | 2.75 g | 2.803 | 0.50 g | 0.500 |
| 5 | 0.50 g | 0.551 | 1.25 g | 1.261 | 3.00 g | 3.011 | 0.25 g | 0.251 |
| 6 | 0.50 g | 0.495 | 1.25 g | 1.267 | 2.95 g | 2.943 | 0.30 g | 0.304 |
| 7 | 0.50 g | 0.522 | 1.25 g | 1.266 | 2.90 g | 2.909 | 0.35 g | 0.353 |
| 8 | 0.50 g | 0.524 | 1.50 g | 1.500 | 2.50 g | 2.510 | 0.50 g | 0.506 |
| 9 | na | | 1.00 g | 1.013 | 3.75 g | 3.770 | 0.25 g | † |
| 10 | na | | 1.00 g | 1.026 | 3.70 g | 3.737 | 0.30 g | † |
| 11 | na | | 1.00 g | 1.005 | 3.65 g | 3.645 | 0.35 g | 0.354 |
| 12 | na | | 1.25 g | 1.246 | 3.25 g | 3.243 | 0.50 g | 0.506 |
| 13 | na | | 1.50 g | 1.518 | 3.00 g | 3.004 | 0.50 g | † |

| | | | | | | | | |
|---|---|---|---|---|---|---|---|---|
| †These solutions were not prepared, *i.e*., no drug added due to success of Samples 11 and 12. | | | | | | | | |

| **Table 2.** | | | | | | | | |
|---|---|---|---|---|---|---|---|---|
| **Sample ID** | **CREMOPHOR® EL, Citric Acid Blend** | | **Methoxylated PEG 350** | | **Vitamin E-TPGS** | | **Paclitaxel** | |
| | *Desired Amount* | *Actual Amount* | *Desired Amount* | *Actual Amount* | *Desired Amount* | *Actual Amount* | *Desired Amount* | *Actual Amount* |
| 14 | 0.50 g | 0.510 g | 1.25 g | 1.262 g | 2.95 g | 2.953 g | 0.30 g | 0.303 g |
| 15 | 0.50 g | † | 1.25 g | † | 2.75 g | † | 0.50 g | † |
| 16 | na | | 1.25 g | 1.252 g | 3.45 g | 3.478 g | 0.30 g | 0.302 g |
| 17 | na | | 1.25 g | 1.254 g | 3.25 g | 3.260 g | 0.50 g | 0.504 g |

| | | | | | | | | |
|---|---|---|---|---|---|---|---|---|
| † not prepared. | | | | | | | | |

The same type of dissolution was observed for methoxy PEG 350 as for DMI; it initially became an opaque dispersion which clarified over time.

All samples were surprisingly able to solubilize the specified amount of paclitaxel added. The addition of 10% CREMOPHOR® EL seemed to retard the dissolution process, but it did not cause any problems preventing final dissolution of all of the paclitaxel.

All of the samples were allowed to cool to room temperature. Upon cooling, all of the solutions appeared to remain intact, *i.e.,* no phase separation or precipitation of paclitaxel was visible. The samples containing 10% CREMOPHOR® EL and 25% DMI (Samples 5-8) appeared non-homogeneous, with Vitamin E-TPGS precipitating out within a light amber solution making two phases apparent. Samples 1-4 and 11, 12, 14, and 16 were opaque semi-solids demonstrating no apparent precipitation of active ingredient.

The ability of the methoxy PEG compounds to solubilize paclitaxel alone was also tested. Formulations were prepared according to Table 3 . Methoxy PEG 350 quickly dissolved both levels of paclitaxel. Methoxy PEG 550 also eventually dissolved the paclitaxel, but it was a slower process.

| **Table 3.** | | | |
|---|---|---|---|
| **Methoxylated PEG 350** | | **Paclitaxel** | |
| *Desired Amount* | *Actual Amount* | *Desired Amount* | *Actual Amount* |
| 0.450 g | 0.456 g | 0.050 g | 0.051 g |
| 0.900 g | 0.901 g | 0.100 g | 0.107 g |

| **Methoxylated PEG 550** | | **Paclitaxel** | |
|---|---|---|---|
| Desired Amount | Actual Amount | Desired Amount | Actual Amount |
| 0.450 g | 0.462 g | 0.050 g | 0.049 g |

### Example 3 - Micellar Solubilization

All 20 ml scintillation vials containing the samples described in Examples 1 and 2 were placed in a 40°C incubator. Plastic transfer pipettes were also placed in the 40°C incubator to minimize congealing during transfer. Each sample was removed and a warmed pipette was utilized to transfer aliquots into three (3) vials and into a number 1 size hard gelatin capsule. See Table 4.

| **Table 4.** | | | | | |
|---|---|---|---|---|---|
| **Sample ID** | **Amount Weighed into Vial 1** | **Amount Weighed into Vial 2** | **Amount Weighed into Vial 3** | **Amount of Water Added to Vial 3** | **Amount Weighed into Gelatin Capsule** |
| 1 | 1.054 g | 1.042 g | 1.006 g | 0.048 g | 0.400 g |
| 2 | 1.085 g | 1.012 g | 0.999g | 0.042 g | 0.442 g |
| 3 | 1.040 g | 1.008 g | 0.996 g | 0.041 g | 0.447 g |
| 4 | 1.020 g | 1.036 g | 1.008 g | 0.041 g | 0.430 g |
| 5 | 1.042 g | 0.997 g | 0.996 g | 0.048 g | 0.431 g |
| 6 | 1.009 g | 1.047 g | 0.996 g | 0.054 g | 0.436 g |
| 7 | 1.017 g | 0.994 g | 1.000 g | 0.045 g | 0.438 g |
| 8 | 1.087 g | 1.013 g | 1.004 g | 0.045 g | 0.412 g |
| 9 | N/A | N/A | N/A | N/A | N/A |
| 10 | N/A | N/A | N/A | N/A | N/A |
| 11 | 1.003g | 0.996g | 1.004g | 0.046g | 0.446g |
| 12 | 1.035g | 1.013g | 1.014g | 0.047g | 0.441g |
| 13 | N/A | N/A | N/A | N/A | N/A |
| 14 | 1.020g | 1.049g | 1.022g | 0.043 g | 0.434 g |
| 15 | N/A | N/A | N/A | N/A | N/A |
| 16 | 1.014 g | 1.013 g | 0.995 g | 0.049 g | 0.463 g |
| 17 | 0.996g | 1.021g | 1.005g | 0.067 g | 0.470 g |

| | | | | | |
|---|---|---|---|---|---|
| N/A = did not prepare designated formula | | | | | |

One vial was stored at ambient room temperature, one vial stored at 40°C, one vial was filled with approximately 1g of sample and approximately 0.05g of water (vortexed and placed at 40°C), and one number 1 size capsule was filled (approximately 15 drops) and capped (stored at ambient room temperature in a capped 1 dram, 14.5 x 45 mm, opticlear vial).

### (Sample ID #1 - 10% CREMOPHOR® EL, 20% DMI, 5% paclitaxel, 65% Vitamin E-TPGS)

| | **Sample Description** | **Observations** |
|---|---|---|
| Rep 1 | Vial 1 RT | opaque, waxy solid, light yellow |
| | Vial 2 40°C | clear, pale yellow liquid |
| | Vial 3 + water | clear, pale yellow liquid (40°C) |
| | Capsule RT | opaque, waxy solid, light yellow |
| Rep 2 | Vial 1 RT | opaque, waxy solid, light yellow |
| | Vial 2 40°C | clear, pale yellow liquid |
| | Vial 3 + water | clear, pale yellow liquid (40°C) |
| | Capsule RT | opaque, waxy solid, light yellow |
| Rep 3 | Vial 1 RT | some phase separation visible |
| | Vial 2 40°C | clear, pale yellow liquid |
| | Vial 3 + water | clear, pale yellow liquid |
| | Capsule RT | opaque, waxy solid, light yellow |

### (Sample ID #2 - 10% CREMOPHOR® EL, 20% DMI, 6% paclitaxel, 64% Vitamin E-TPGS)

| | **Sample Description** | **Observations** |
|---|---|---|
| Rep 1 | Vial 1 RT | opaque, waxy solid, light yellow |
| | Vial 2 40°C | clear, pale yellow liquid |
| | Vial 3 + water | clear, pale yellow liquid (40°C) |
| | Capsule RT | opaque, waxy solid, light yellow |
| Rep 2 | Vial 1 RT | opaque, waxy solid, light yellow |
| | Vial 2 40°C | clear, a few strands of shiny particulate |
| | Vial 3 + water | pale yellow liquid with a few strands of shiny ppt (40°C) |
| | Capsule RT | opaque, waxy solid, light yellow |
| Rep 3 | Vial 1 RT | opaque, waxy solid, light yellow |
| | Vial 2 40°C | clear, few strands of shiny ppt |
| | Vial 3 + water | clear, pale yellow liquid with few strands of ppt |
| | Capsule RT | opaque, waxy solid, light yellow |

### (Sample ID #3 - 10% CREMOPHOR® EL, 20% DMI, 7% paclitaxel, 63% Vitamin E-TPGS)

| | **Sample Description** | **Observations** |
|---|---|---|
| Rep 1 | Vial 1 RT | opaque, waxy solid, light yellow |
| | Vial 2 40°C | clear, pale yellow liquid |
| | Vial 3 + water | clear, pale yellow liquid (40°C) |
| | Capsule RT | opaque, waxy solid, light yellow |
| Rep 2 | Vial 1 RT | opaque, waxy solid, light yellow |
| | Vial 2 40°C | clear, pale yellow liquid |
| | Vial 3 + water | clear, pale yellow liquid (40°C) |
| | Capsule RT | opaque, waxy solid, light yellow |

### (Sample ID #4 - 10% CREMOPHOR® EL, 20% DMI, 10% paclitaxel, 60% Vitamin E-TPGS)

| | **Sample Description** | **Observations** |
|---|---|---|
| Rep 1 | Vial 1 RT | multiple phases visible, striation |
| | Vial 2 40°C | clear, pale yellow liquid |
| | Vial 3 + water | clear, pale yellow liquid (40°C) |
| | Capsule RT | apparent phase separation |
| Rep 2 | Vial 1 RT | some striation at bottom, otherwise waxy mass |
| | Vial 240°C | clear, pale yellow liquid |
| | Vial 3 + water | clear, pale yellow liquid (40°C) |
| | Capsule RT | apparent multiple phases |

### (Sample ID #5 - 10% CREMOPHOR® EL, 25% DMI, 5% paclitaxel, 60% Vitamin E-TPGS)

| | **Sample Description** | **Observations** |
|---|---|---|
| Rep 1 | Vial 1 RT | opaque, waxy solid, light yellow |
| | Vial 2 40°C | clear, pale yellow liquid |
| | Vial 3 + water | clear, pale yellow liquid (40°C) |
| | Capsule RT | opaque, waxy solid, light yellow |
| Rep 2 | Vial 1 RT | opaque, waxy solid, light yellow |
| | Vial 2 40°C | light yellow liquid with some shiny crystalline ppt |
| | Vial 3 + water | light yellow liquid with some shiny, crystalline ppt (40°C) |
| | Capsule RT | opaque, waxy solid, light yellow |
| Rep 3 | Vial 1 RT | definite phase separation, light yellow, clear and solid off-white wax |
| | Vial 2 40°C | light yellow liquid with some shiny ppt |
| | Vial 3 + water | light yellow liquid with some shiny ppt |
| | Capsule RT | multiple phases visible, intermingled |

### (Sample ID #6 - 10% CREMOPHOR® EL, 25% DMI, 6% paclitaxel, 59% Vitamin E-TPGS)

| | **Sample Description** | **Observations** |
|---|---|---|
| Rep 1 | Vial 1 RT | apparent phase separation, striated |
| | Vial 2 40°C | clear, pale yellow liquid |
| | Vial 3 + water | clear, pale yellow liquid (40°C) |
| | Capsule RT | apparent phase separation |
| Rep 2 | Vial 1 RT | opaque, waxy solid, off-white |
| | Vial 2 40°C | light yellow liquid with some shiny crystalline ppt (40°C) |
| | Vial 3 + water | light yellow liquid with some shiny, crystalline ppt (40°C) |
| | Capsule RT | opaque, waxy solid, off-white |
| Rep 3 | Vial 1 RT | definite phase separation, light yellow, clear and solid off-white wax |
| | Vial 2 40°C | light yellow liquid with some shiny ppt |
| | Vial 3 + water | light yellow liquid with some shiny ppt |
| | Capsule RT | multiple phases visible, intermingled |

### (Sample ID #7 - 10% CREMOPHOR® EL, 25% DMI, 7% paclitaxel, 58% Vitamin E-TPGS)

| | **Sample Description** | **Observations** |
|---|---|---|
| Rep 1 | Vial 1 RT | apparent phase separation, striated |
| | Vial 240°C | clear, pale yellow liquid |
| | Vial 3 + water | clear, pale yellow liquid (40°C) |
| | Capsule RT | apparent phase separation |
| Rep 2 | Vial 1 RT | congealed with striations |
| | Vial 2 40°C | light yellow liquid with some shiny crystalline ppt |
| | Vial 3 + water | light yellow liquid with some shiny, crystalline ppt (40°C) |
| | Capsule RT | congealed with striations |
| Rep 3 | Vial 1 RT | multiphase appearance |
| | Vial 2 40°C | light yellow, clear liquid with shiny ppt |
| | Vial 3 + water | light yellow, clear liquid with shiny ppt |
| | Capsule RT | multiphase appearance |

### (Sample ID #8 - 10% CREMOPHOR® EL, 25% DMI, 10% paclitaxel, 55% Vitamin E-TPGS)

| | **Sample Description** | **Observations** |
|---|---|---|
| Rep 1 | Vial 1 RT | apparent phase separation, striated |
| | Vial 2 40°C | clear, pale yellow liquid |
| | Vial 3 + water | clear, pale yellow liquid (40°C) |
| | Capsule RT | completely clear solution |
| Rep 2 | Vial 1 RT | liquid and solid phases visible, liquid on top |
| | Vial 2 40°C | light yellow liquid with some shiny crystalline ppt |
| | Vial 3 + water | light yellow liquid with some shiny, crystalline ppt (40°C) |
| | Capsule RT | liquid and solid phases visible |
| Rep 3 | Vial 1 RT | multiphase system in appearance |
| | Vial 2 40°C | light yellow, clear liquid with shiny ppt |
| | Vial 3 + water | light yellow, clear liquid with shiny ppt |
| | Capsule RT | clear, pale yellow liquid |

### (Sample ID #11 - 20% DMI, 7% paclitaxel, 73% Vitamin E-TPGS)

| | **Sample Description** | **Observations** |
|---|---|---|
| Rep 1 | Vial 1 RT | opaque, waxy solid, light yellow |
| | Vial 2 40°C | clear, pale yellow liquid |
| | Vial 3 + water | clear, pale yellow liquid (40°C) |
| | Capsule RT | opaque, waxy solid, light yellow |
| Rep 2 | Vial 1 RT | opaque, waxy solid, light yellow |
| | Vial 240°C | clear, pale yellow liquid |
| | Vial 3 + water | clear, pale yellow liquid (40°C) |
| | Capsule RT | opaque, waxy solid, light yellow |

### (Sample ID #12-25% DMI, 10% paclitaxel, 70% Vitamin E-TPGS)

| | **Sample Description** | **Observations** |
|---|---|---|
| Rep 1 | Vial 1 RT | apparent phase separation, striated |
| | Vial 2 40°C | clear, pale yellow liquid |
| | Vial 3 + water | clear, pale yellow liquid (40°C) |
| | Capsule RT | apparent phase separation |
| Rep 2 | Vial 1 RT | semi-solid with striations |
| | Vial 2 40°C | clear, pale yellow liquid |
| | Vial 3 + water | clear, pale yellow liquid (40°C) |
| | Capsule RT | semi-solid with striations |

### (Sample ID #14 - 10% CREMOPHOR® EL, 25% MPEG, 6% paclitaxel, 59% Vitamin E-TPGS)

| | **Sample Description** | **Observations** |
|---|---|---|
| Rep 1 | Vial 1 RT | opaque, off-white waxy solid |
| | Vial 2 40°C | clear, pale yellow liquid |
| | Vial 3 + water | clear, pale yellow liquid (40°C) |
| | Capsule RT | opaque, off-white waxy solid |
| Rep 2 | Vial 1 RT | opaque, off-white waxy solid |
| | Vial 2 40°C | clear, pale yellow liquid |
| | Vial 3 + water | clear, pale yellow liquid (40°C) |
| | Capsule RT | opaque, off-white waxy solid |

To test the micellar solution capability of the above samples, Samples 4, 8, and 12 were added to water. Formula composition 4 = 10% CREMOPHOR® EL, 25% DMI, 10% paclitaxel, and 55% Vitamin E-TPGS. Formula composition 8 = 10% CREMOPHOR® EL, 30% DMI, 10% paclitaxel, and 50% Vitamin E-TPGS. Formula composition 12 = 25% DMI, 10% paclitaxel, and 65% Vitamin E-TPGS. Five grams of water were added into 20 ml scintillation vials. 3 separate vials were prepared, each containing approximately 5 ml of water, and to each approximately 5 drops of one of formulations 4, 8, and 12 were added. Each vial was agitated to see if any precipitation occurred. Formulations 4 and 12 yielded clear solutions upon agitation. Formulation 8 immediately yielded a slightly turbid solution. This visual condition suggests that the portions of DMI and CREMOPHOR® EL may be disrupting the assembly of the micelles. It is believed that the micelles are formed as Vitamin E-TPGS hydrates to form a cubic phase structure. The drug is encapsulated and eventually released into solution as this process progresses. One explanation for this is that the quantities of DMI/CREMOPHOR® EL present cause the immediate dissolution of Vitamin E-TPGS, inhibiting the excipient from forming a transient cubic phase.

Both Formulations 4 and 12 became clear with many bubbles at the surface. The drops of formulation became gelatinous (process of hydrating) and then slowly dissolved. The final solutions were clear. After twelve hours, the aqueous solution containing 5 drops of Formulation 4 produced a thin film of precipitated material on the bottom of the vial. Formulation 12 remained clear with no evidence of precipitated material after twelve hours.

### Example 4 - Taxane-Containing Gel Formulations

1% (0.05g) paclitaxel was dissolved in 4.75g of dimethylisosorbide. 0.20g of KLUCEL® HF was sprinkled over the surface while the solution was rapidly stirred. This was carried out at room temperature using 20 ml scintillation vials containing a one-half inch egg-shaped stir bar placed on a Coming stir plate. The solution was clear and fluid upon completion. After sitting for approximately 30 minutes, the solution gelled.

### Example 5 - Semi-Solid Formulations for Oral Administration

A container of Vitamin E-TPGS was placed in a 40°C incubator, to liquefy the excipient. Plastic transfer pipettes were also warmed in the incubator to transfer the warmed excipient. Each formulation was again prepared in a 20 ml scintillation vial containing a one-half inch egg-shaped stir bar: The vial and stir bar were initially tared. Warmed Vitamin E-TPGS was dispensed to each tared vial using a warmed plastic transfer pipette. Each vial was subsequently tared and the desired amount of DMI was added using a plastic transfer pipette. Each vial was subsequently tared and the desired amount of PEG 4600 flake was added to two of the three vials. See Table 5 (Samples 19 and 20). Two scintillation vials (Samples 18 and 19) were placed on a stir/hotplate (the formulations containing PEG 4600 were given slightly increased heat as compared to Sample 18 to aid in melting the thickener). Small weighing canoes were tared on a balance and paclitaxel was transferred until the desired weight was achieved. Large clumps were broken apart easily with the stainless steel spatula. The paclitaxel was slowly added to the warmed, stirring excipients of the Samples 18 and 19.

| **Table 5.** | | | | | | | | |
|---|---|---|---|---|---|---|---|---|
| **Sample ID** | **PEG 4600 Flake** | | **DMI** | | **Vitamin E-TPGS** | | **Taxanes** | |
| | *Desired Amount* | *Actual Amount* | *Desired Amount* | *Actual Amount* | *Desired Amount* | *Actual Amount* | *Desired Amount* | *Actual Amount* |
| 18 | na | - | 1.00 g | 0.993 g | 3.50 g | 3.505 g | 0.50 g | 0.505 g |
| 19 | 0.25 g | 0.259 g | 1.00 g | 0.996 g | 3.25 g | 3.254 g | 0.50 g | 0.502 g |
| 20 | 0.25 g | 0.259 g | 1.25 g | 1.252 g | 3.00 g | 3.015 g | 0.50 g | 0.501 g |

The bulk drug again dissipated quickly and the solutions clarified rapidly.

The same procedure outlined above was used to prepare the final formulation of Sample 20.

Formulations were allowed to cool and were stored at ambient room temperature. To further characterize Formulations 18-20 they were subjected to the same procedures outlined in Example 3. Formulations 18-20 were warmed to liquefy and allow easier transfer. Transfer pipettes were also warmed. Three vials were prepared for each of Formulations 18-20, a total of nine vials, each vial containing 1g of formula. In addition, approximately 15 drops of each formulation were filled into a number 1 hard gel capsule. To the third vial of each formula, one drop of water was added and the sample vortexed. See Table 6.

| **Table 6.** | | | | | |
|---|---|---|---|---|---|
| **Sample ID** | **Amount Weighed into Vial 1** | **Amount Weighed into Vial 2** | **Amount Weighed into Vial 3** | **Amount of Water Added to Vial 3** | **Amount Weighed into Gelatin Capsule** |
| 18 | 1.023 g | 1.057 g | 1.007 g | 0.058 g | 0.445 g |
| 19 | 1.013 g | 1.038 g | 1.000 g | 0.055 g | 0.433 g |
| 20 | 0.999 g | 1.016 g | 1.002 g | 0.052 g | 0.418 g |

Five drops of each warmed solution were added to approximately 5 ml of water. At this time, the behavior of Samples 16 and 17 were also tested by introduction to approximately 5 ml of water.

Formulation 16 - Approximately 5g (~5 ml) of water were transferred to a 20ml scintillation vial. 5 drops of formulation were added (~150 mg) and the mixture agitated slightly and continuously.

The drops of formulation congealed immediately upon striking the surface of the water (~20°C). The mass sank to the bottom and at first seemed to be just a precipitated globule of product. However, after 1 to 2 minutes of mild agitation, a translucent, crystal clear erosion layer could be seen around the entire aqueous exposed surface of the mass. Eventually, the entire mass dissolved as the erosion layer receded into the interior of the mass. The translucent, clear erosion layer appeared to be about 2 mm in thickness. The center remained opaque and solid until the erosion layer consumed the entire mass. The resulting solution was clear with a significant number of surfactant bubbles at the surface.

Formulation 17 - This formulation behaved exactly the same as Formulation 16. The only difference between the two was drug loading, 6% for 16, 10% for 17.

Formulation 18 (20% DMI, 10% paclitaxel, 70% TPGS) - Five drops into 5 ml water; slightly agitated solution of formulation congealed upon striking the cool water (~20°C) and slowly dissolved into the aqueous solution. The mass (entire mass) quickly became translucent and gelatinous in appearance. Eventually all of the mass dissolved, yielding an aqueous solution with no apparent precipitated drug.

Formulation 19 (5% PEG 4600, 20% DMI, 10% paclitaxel, 65% TPGS) - Five drops into 5 ml water; slightly agitated. This formulation behaved essentially the same as Formulation 18. However, upon striking the cool water (~20°C), the mass seemed to elongate and disperse in the aqueous fraction. Striations were observed within the mass, which rapidly became translucent. The mass appeared to be like a bunch of spaghetti noodles structured together and wrapped around each other. The mass spread out along the bottom of the vial and appeared much more fluid than when PEG 4600 is absent in the formulation. Eventually, the mass dissolved, leading again to a clear solution with many bubbles at the surface.

Formulation 20 (5% PEG 4600, 25% DMI, 10% paclitaxel, 60% TPGS) - This formulation behaved just like Formulation 19.

### (Sample ID #16 - 25% MPEG, 6% paclitaxel, 69% Vitamin E-TPGS)

| | **Sample Description** | **Observations** |
|---|---|---|
| Rep 1 | Vial 1 RT | opaque, off-white waxy solid |
| | Vial 240°C | clear, pale yellow liquid |
| | Vial 3 + water | clear, pale yellow liquid (40°C) |
| | Capsule RT | opaque, off-white waxy solid |
| Rep 2 | Vial 1 RT | opaque, off-white waxy solid |
| | Vial 240°C | clear, pale yellow liquid |
| | Vial 3 + water | clear, pale yellow liquid (40°C) |
| | Capsule RT | opaque, off-white waxy solid |

### (Sample ID #17 - 25% MPEG, 10% paclitaxel, 65% Vitamin E-TPGS)

| | **Sample Description** | **Observations** |
|---|---|---|
| Rep 1 | Vial 1 RT | opaque, off-white waxy solid |
| | Vial 2 40°C | clear, pale yellow liquid |
| | Vial 3 + water | clear, pale yellow liquid (40°C) |
| | Capsule RT | opaque, off-white waxy solid |
| Rep 2 | Vial 1 RT | opaque, off-white waxy solid |
| | Vial 2 40°C | clear, pale yellow liquid |
| | Vial 3 + water | clear, pale yellow liquid (40°C) |
| | Capsule RT | opaque, off-white waxy solid |

### (Sample ID #18 - 20% DMI, 10% paclitaxel, 70% Vitamin E-TPGS)

| | **Sample Description** | **Observations** |
|---|---|---|
| Rep 1 | Vial 1 RT | opaque, waxy solid, light yellow |
| | Vial 2 40°C | clear, pale yellow liquid |
| | Vial 3 + water | clear, pale yellow liquid (40°C) |
| | Capsule RT | opaque, waxy solid, light yellow |
| Rep 2 | Vial 1 RT | opaque, waxy solid, light yellow |
| | Vial 2 40°C | clear, pale yellow liquid |
| | Vial 3 + water | clear, pale yellow liquid (40°C) |
| | Capsule RT | opaque, waxy solid, light yellow |

### (Sample ID #19 - 20% DMI, 5% PEG 4600, 10% paclitaxel, 65% Vitamin E-TPGS)

| | **Sample Description** | **Observations** |
|---|---|---|
| Rep 1 | Vial 1 RT | opaque, off-white waxy solid |
| | Vial 2 40°C | material ppt on bottom, probably PEG 4600 |
| | Vial 3 + water | clear, pale yellow liquid (40°C) |
| | Capsule RT | opaque, off-white waxy solid |
| Rep 2 | Vial 1 RT | opaque, off-white waxy solid |
| | Vial 2 40°C | material ppt on bottom, probably PEG 4600 |
| | Vial 3 + water | clear, pale yellow liquid (40°C) |
| | Capsule RT | opaque, off-white waxy solid |
| Rep 3 | Vial 1 RT | opaque, off-white waxy solid somewhat rigid |
| | Vial 2 40°C | definite phase separation |
| | Vial 3 + water | clear, yellow liquid with a lot of ppt |
| | Capsule RT | opaque, off-white waxy solid |

### (Sample ID #20 - 25% DMI, 5% PEG 4600, 10% paclitaxel, 60% Vitamin E-TPGS)

| | **Sample Description** | **Observations** |
|---|---|---|
| Rep 1 | Vial 1 RT | opaque, off-white waxy solid |
| | Vial 2 40°C | material ppt on bottom, probably PEG 4600 |
| | Vial 3 + water | clear, pale yellow liquid (40°C) |
| | Capsule RT | opaque, off-white waxy solid |
| Rep 2 | Vial 1 RT | opaque, off-white waxy solid |
| | Vial 2 40°C | material ppt on bottom, probably PEG 4600 |
| | Vial 3 + water | clear, pale yellow liquid (40°C) |
| | Capsule RT | opaque, off-white waxy solid |
| Rep 3 | Vial 1 RT | opaque, off-white waxy solid granular in appearance |
| | Vial 2 40°C | definite phase separation |
| | Vial 3 + water | clear, pale yellow liquid |
| | Capsule RT | opaque, off-white waxy solid |

Formulations 19 and 20, containing 5% PEG 4600, exhibited two main characteristics: (1) precipitation was visible in those formulations that contained 5% PEG 4600 and were kept at 40°C; and (2) no precipitation was visible in those solutions containing 5% PEG 4600, kept at 40°C, and which contained 5% added water. These observations indicated that added water actually helps maintain the PEG 4600 in solution. The desired thickening effect of the PEG 4600 thus may require water to be present in the matrix to maintain a homogeneous mass.

### Example 6 - Vitamin E-TPGS Taxane Formulations Using PEG 300 as a Co-Solvent

Due to the success of the methoxypolyethylene glycol 350 formulations, PEG 300 was pursued as another possible candidate for a co-solvent in the Vitamin E-TPGS formulations. PEG 400 has shown some success in solubilizing taxanes. However, PEG 300 would be particularly desirable as a co-solvent because it is already approved for oral use in prescription drugs.

Vitamin E-TPGS was warmed in a 40°C oven and once it was fluid, the desired quantity was transferred using a plastic transfer pipette into a 20 ml scintillation vial containing a stir bar. To the liquidified Vitamin E-TPGS the desired amount of PEG 300 was added to the vial. The PEG 300/Vitamin E-TPGS mixture was stirred while being warmed on the stir/hotplate. Paclitaxel was added slowly, with stirring, to the warmed PEG 300/Vitamin E-TPGS mixture. The mixture was stirred while warm until all of the paclitaxel dissolved. The formulation was then distributed into three (3) vials and one (1) hard gelatin capsule, as described above. One vial was stored at room temperature along with the capsule; the other two vials were placed in a 40°C oven.

### (Sample ID No. 21 - 25% PEG 300, 65% Vitamin E TPGS, 10% paclitaxel)

| | **Sample Description** | **Observations** |
|---|---|---|
| Rep 1 | Vial 1 RT | opaque, waxy solid, light yellow |
| | Vial 2 40°C | clear, light yellow solution |
| | Vial 3 + Water | clear, light yellow solution (40°C) |
| | Capsule RT | opaque, waxy solid, light yellow |

The samples were clear, amber yellow solutions while being warmed. The vial which was allowed to cool to room temperature became a yellow, waxy solid mass. While still warm, about 5 drops of the warmed formulation was transferred and added to about 5 ml of water and agitated. The mass initially solidified, but subsequently slowly dissolved into the aqueous layer. Eventually all of the mass dissipated into the water, and bubbles were apparent on the surface of the water. All of the paclitaxel was dissolved without any visually apparent precipitate.

### Example 7 - Preparation of a Vitamin E-TPGS Formulation Containing Paclitaxel Suitable for Parenteral Delivery

The current commercially available formulation of paclitaxel is provided as a 50/50 mixture CREMOPHOR® EL/ethanol. Some of the formulations which have been described in the examples above are not optimally suited for parenteral delivery due to their solid or semi-solid consistency. This physical state does not lend itself well to sterilization technology. Accordingly, it was desirable to develop a liquid formulation particularly suitable for sterile filtration.

1.016g of ethyl alcohol was transferred to a 20 ml scintillation vial containing a stir bar. 1.023g of Vitamin E-TPGS was added to the vial. The solution was warmed slightly to facilitate the dissolution of the Vitamin E-TPGS. Upon dissolution of Vitamin E-TPGS, 0.205g of paclitaxel was added with stirring. The paclitaxel dissolved quickly. To determine if the high level of co-solvent disturbs the ability of Vitamin E-TPGS to capture paclitaxel into micelles, approximately 6-8 drops of formulation were transferred to about 5 ml of water. Upon addition of the formulation to the water, the mixture was slightly agitated, which yielded a turbid solution. This turbidity is indicative of microprecipitation, suggesting that the cubic phase of Vitamin E-TPGS had been disrupted, thereby allowing the paclitaxel to precipitate when added to water. However, the turbid solution was not discarded, but was allowed to sit for a period of time. Surprisingly, the microparticulate dissipated and the solution appeared very clear with some bubbles on the surface of the water. This demonstrated that it is not necessary that the active component be captured and internalized during dissolution of the Vitamin E-TPGS into water. Encapsulation of paclitaxel can occur over time, and is driven by partitioning of paclitaxel into the dynamic micelles present in the aqueous matrix.

However, the ethanol/Vitamin E-TPGS formulation maintained a slight turbidity. This observation suggested that paclitaxel may not be completely soluble in this composition, or that Vitamin E-TPGS may not be completely miscible in ethanol.

To overcome or avoid the possible physical incompatibility between ethanol and Vitamin E-TPGS many of the variables were adjusted.

2.255g of ethanol, 0.020g of citric acid, and 0.25g of water were combined in a 20 ml scintillation vial. The mixture was agitated slightly to dissolve the citric acid. To the ethanol and citric acid aqueous mixture, 2.542g of Vitamin E-TPGS was added, warmed, and stirred. The Vitamin E-TPGS liquefied at 40°C. To this warmed, stirred solution, 0.053g of paclitaxel was added with continued stirring.

The solution was fluid and appeared very clear. There was absolutely no turbidity from either undissolved taxanes or Vitamin E-TPGS.

To test the ability of this formulation to capture the taxane into micelles, 5 drops of the formulation was transferred to 5 ml of water. No turbidity was observed. Only small pieces of gelatinous material could be seen at first, which soon dissipated to yield a crystal clear solution. This formulation which was added to the water was allowed to cool to room temperature. Upon cooling, the solution was still clear. After being allowed to sit for at least 24 hours, the aqueous solution still remained clear.

### Example 8 - Production and Characterization of Additional Parenteral Formulations

Having prepared a working formulation as shown in Example 7, additional formulations were prepared:
20% ethanol, 80% TPGS;
40% ethanol, 60% TPGS;
50% ethanol, 50% TPGS;
62.5% ethanol, 37.5% TPGS; and
75% ethanol, 25% TPGS.

To each of these formulations paclitaxel was added in the amounts of 6 mg/ml, 10 mg/ml, 20 mg/ml, and 50 mg/ml.

Citric acid was weighed into each of five 20 ml scintillation vials to which ethanol was added using a plastic transfer pipette. The mixture was agitated to dissolve the citric acid. Vitamin E-TPGS was liquefied in a 40°C oven and then carefully poured into each scintillation vial. The vials were warmed slightly to liquefy the Vitamin E-TPGS and to accelerate dissolution into the ethanol/citric acid co-solvent mixture. See Table 7. The vials were shaken until the mixture appeared uniform. All of the solutions were allowed to cool to room temperature. Upon cooling, each of the above formulations were then distributed into 4 individual 20 ml scintillation vials, so that each vial contained approximately 4g of the mixture. To each of these vials, 24, 40, 80, or 200 mg of paclitaxel was added. See Table 8. Each formulation was agitated until the taxane dissolved or reached an equilibrium solubility.

| **Table 7.** | | | | | | |
|---|---|---|---|---|---|---|
| **Sample ID** | **Ethanol** | | **Citric Acid** | | **Vitamin E-TPGS** | |
| | *Desired Amount* | *Actual Amount* | *Desired Amount* | *Actual Amount* | *Desired Amount* | *Actual Amount* |
| A (20%) | 3.60 g | 3.604 g | 0.036 g | 0.036 g | 14.40 g | 14.402 g |
| B (40%) | 7.20 g | 7.205 g | 0.036 g | 0.036 g | 10.80 g | 10.805 g |
| C (50%) | 9.00 g | 9.010 g | 0.036 g | 0.038 g | 9.00 g | 9.024 g |
| D (62.5%) | 11.25 g | 11.252 g | 0.036 g | 0.038 g | 6.75 g | 6.772 g |
| E (75%) | 13.50 g | 13.499 g | 0.036 g | 0.037 g | 4.50 g | 4.509 g |

| **Table 8.** | | | | |
|---|---|---|---|---|
| **Sample ID** | **Amount of Formulation Weighed into Vial** | | **Amount of Paclitaxel Weighed into Vial** | |
| | *Desired Amount* | *Actual Amount* | *Desired Amount* | *Actual Amount* |
| A-1 | 4.00 g | 4.010 g | 0.024 g | 0.024 g |
| A-2 | 4.00 g | 4.013 g | 0.040 g | 0.041 g |
| A-3 | 4.00 g | 4.026 g | 0.080 g | 0.080 g |
| A-4 | 4.00 g | 4.027 g | 0.200 g | 0.201 g |
| B-1 | 4.00 g | 4.018 g | 0.024 g | 0.024 g |
| B-2 | 4.00 g | 4.021 g | 0.040 g | 0.041 g |
| B-3 | 4.00 g | 4.010 g | 0.080 g | 0.080 g |
| B-4 | 4.00 g | 4.004 g | 0.200 g | 0.201 g |
| C-1 | 4.00 g | 4.000 g | 0.024 g | 0.024 g |
| C-2 | 4.00 g | 4.001 g | 0.040 g | 0.040 g |
| C-3 | 4.00 g | 4.018 g | 0.080 g | 0.081 g |
| C-4 | 4.00 g | 4.006 g | 0.200 g | 0.199 g |
| D-1 | 4.00 g | 4.008 g | 0.024 g | 0.024 g |
| D-2 | 4.00 g | 4.005 g | 0.040 g | 0.042 g |
| D-3 | 4.00 g | 4.028 g | 0.080 g | 0.080 g |
| D-4 | 4.00 g | 4.003 g | 0.200 g | 0.200 g |
| E-1 | 4.00 g | 4.003 g | 0.024 g | 0.024 g |
| E-2 | 4.00 g | 4.040 g | 0.040 g | 0.041 g |
| E-3 | 4.00 g | 4.014 g | 0.080 g | 0.081 g |
| E-4 | 4.00 g | 4.009 g | 0.200 g | 0.202 g |

Solution E-4 (75% ethanol, 25% TPGS, 50 mg/ml paclitaxel) persisted in a state of turbidity, which suggested that the taxane had exceeded its solubility in this mixture. It is unlikely that the Vitamin E-TPGS was responsible for the turbidity, as it would precipitate out as small, star-like masses.

Solutions A-1 through A-4 (20% ethanol, 80% TPGS) all were viscous.

A CREMOPHOR® EL control preparation was made to provide a standard against which the Vitamin E-TPGS formulations could be measured. The same procedures used above to produce the Vitamin E-TPGS formulations were used to produce the CREMOPHOR® EL formulation, except that no warming was required, as CREMOPHOR® EL is a liquid at room temperature.

Formulations B-1 through E-3 were diluted five-fold and twenty-fold in water to monitor physical stability of the TPGS micelles in the presence of various amounts of ethanol and paclitaxel. (The A- series was not pursued further at this point, as precipitation of TPGS was apparent in 2 of the 4 preparations. Further, preparation E-4 was omitted, as the paclitaxel never completely dissolved and remained in the form of a microparticulate.) 4g (5-fold dilution) or 9.5g (20-fold dilution) of water was transferred to individual 20 ml scintillation vials. 1g of the formulations was added to the vials containing the 4g of water, and 0.5g of the formulations was added to the vials containing 9.5g of water. The solutions were agitated and visually inspected. The diluted solutions were then monitored for duration of physical stability.
B series (40% ethanol) - formulation solidified into a gelatinous mass which subsequently dissolved and dissipated over time; agglomerated.
C series (50% ethanol) - formulations dispersed very quickly with small fragments of gelatinous matrix visible. However, dissolution occurred very quickly.
D series (62.5% ethanol) - formulations dispersed immediately when contacting water. Dissolution occurred almost instantaneously.
E series (75% ethanol) - same as D series above.

Absolutely no turbidity was observed for any of the formulations upon introduction to the water. This observation was true despite compositional differences and the 6, 10, 20, or 50 mg/ml of paclitaxel present.

The rate at which turbidity or precipitation occurred in the solutions appears to be comparable to the behavior seen for the CREMOPHOR® EL/ethanol (5g EtOH, 0.0205g Citric Acid, 5g of CREMOPHOR® EL, and 0.060g paclitaxel) control sample when diluted into water.

All of the above solutions were visually inspected on an ongoing time basis to determine the duration of physical stability when in contact with water. See Table 9. At the 24-hour time interval, all but the solutions containing 50% ethanol:50% Vitamin E-TPGS (6 or 10 mg/ml taxanes) displayed precipitation when diluted at the 5-fold level. All of the 20-fold dilution samples retained the characteristics observed at 12 hours. The precipitate was visualized by swirling the vials in a clockwise fashion.

| **Table 9.** | | | | | | | | | | | | |
|---|---|---|---|---|---|---|---|---|---|---|---|---|
| **Sample ID** | **1 hr** | **2 hr** | **3 hr** | **4 hr** | **5 hr** | **6 hr** | **7 hr** | **8 hr** | **9 hr** | **10 hr** | **11 hr** | **12 hr** |
| B-1(5x) | x | x | x | x | x | x | x | x | x | x | x | x |
| B-2(5x) | x | x | x | x | x | x | x | x | x | x | x | x |
| B-3(5x) | x | x | x | x | x | x | x | x | x | x | x | x |
| B-4(5x) | x | x | ppt | | | | | | | | | |
| C-1(5x) | x | x | x | x | x | x | x | x | x | x | x | x |
| C-2(5x) | x | x | x | x | x | x | x | x | x | x | x | x |
| C-3(5x) | x | x | x | x | x | x | x | ppt | | | | |
| C-4(5x) | x | ppt | | | | | | | | | | |
| D-1(5x) | x | x | x | x | x | x | x | x | x | x | x | x |
| D-2(5x) | x | x | x | x | x | x | x | x | x | x | x | x |
| D-3(5x) | x | x | ppt | | | | | | | | | |
| D-4(5x) | x | ppt | | | | | | | | | | |
| E-1(5x) | x | x | x | x | x | x | x | x | x | x | x | x |
| E-2(5x) | x | x | x | ppt | | | | | | | | |
| E-3(5x) | ppt | | | | | | | | | | | |
| E-4(5x) | na | | | | | | | | | | | |
| Control | x | x | x | x | x | x | x | x | x | x | x | x |

| | | | | | | | | | | | | |
|---|---|---|---|---|---|---|---|---|---|---|---|---|
| Key: ppt = visual precipitate or particulate; x = clear solution. | | | | | | | | | | | | |

| **Table 10.** | | | | | | | | | | | | |
|---|---|---|---|---|---|---|---|---|---|---|---|---|
| **Sample ID** | **1 hr** | **2 hr** | **3 hr** | **4 hr** | **5 hr** | **6 hr** | **7 hr** | **8 hr** | **9 hr** | **10 hr** | **11 hr** | **12 hr** |
| B-1(20x) | x | x | x | x | x | x | x | x | x | x | x | x |
| B-2(20x) | x | x | x | x | x | x | x | x | x | x | x | x |
| B-3(20x) | x | x | x | x | x | x | x | x | x | x | x | x |
| B-4(20x) | x | x | x | x | x | x | ppt | | | | | |
| C-1(20x) | x | x | x | x | x | x | x | x | x | x | x | x |
| C-2(20x) | x | x | x | x | x | x | x | x | x | x | x | x |
| C-3(20x) | x | x | x | x | x | x | x | x | x | x | x | x |
| C-4(20x) | x | x | x | x | x | ppt | | | | | | |
| D-1(20x) | x | x | x | x | x | x | x | x | x | x | x | x |
| D-2(20x) | x | x | x | x | x | x | x | x | x | x | x | x |
| D-3(20x) | x | x | x | x | x | x | x | x | x | x | x | ppt |
| D-4(20x) | x | x | x | ppt | | | | | | | | |
| E-1(20x) | x | x | x | x | x | x | x | x | x | x | x | x |
| E-2(20x) | x | x | x | x | x | x | x | x | x | x | x | x |
| E-3(20x) | x | x | x | x | ppt | | | | | | | |
| E-4(20x) | na | | | | | | | | | | | |
| Control | x | x | x | x | x | x | x | x | x | x | x | x |

| | | | | | | | | | | | | |
|---|---|---|---|---|---|---|---|---|---|---|---|---|
| Key: ppt = visual precipitate or particulate; x = clear solution. | | | | | | | | | | | | |

### Example 9 - Oral Feasibility PK

A preferred formulation for the oral delivery of paclitaxel was evaluated for safety in mammals using dogs. The formulation was as follows:

| | | |
|---|---|---|
| Vitamin E TPGS | Eastman | 70% |
| Dimethylisosorbide | ICI | 20% |
| paclitaxel | NBT | 10% |
| Citric acid (anhydrous) Sigma | 2 mg/g | |

and was hand filled into single 0 hard gelatin capsules to a dose of approximately 34 mg/capsule.

Six male beagles, all approximately six months old, were given paclitaxel capsules at doses from 3 to 11 milligram of drug per kilogram of body weight. Capsules were inserted into the esophagus and followed with a brief squirt of water and the animals held until swallowing was observed. Fourteen blood samples - 16 hours predose through 48 hours postdose - were drawn from each animal and the plasma analyzed for paclitaxel by a sensitive and specific HPLC method. Five of the six animals had detectable levels of intact drug following dosing. The plasma level data have been reduced by noncompartmental analysis and the results are presented in Table 11.

| **Table 11.** | | | | | | | |
|---|---|---|---|---|---|---|---|
| Model-independent bioavailability summary | | | | | | | |
| Animal | Mass, kg | Caps given | Dose, mg/kg | Cₘₐₓ¹, ng/mL | AUC², nghr/mL | ¹ₘₐₓ³, hr | ¹ₗₐₛₜ⁴, hr |
| PXF-8 | 10.4 | 1 | 3.23 | 17 | 194 | 1 | 12 |
| OEF-8 | 10.3 | 1 | 3.27 | 97 | 187 | 1 | 8 |
| QEF-8 | 10.2 | 1 | 3.30 | nd⁵ | nd | nd | nd |
| IOF-8 | 14.8 | 3 | 6.88 | 92 | 135 | 0.5 | 6 |
| FJF-8 | 13.4 | 3 | 7.66 | 108 | 202 | 1 | 8 |
| OLF-8 | 9.3 | 3 | 11.14 | 63 | 189 | 1 | 8 |

| | | | | | | | |
|---|---|---|---|---|---|---|---|
| These data confirm the systemic availability of paclitaxel following oral administration of this formulation. | | | | | | | |
| ¹Maximum paclitaxel concentration observed in the plasma | | | | | | | |
| ²Calculated as the sum of trapezoids | | | | | | | |
| ³Time of maximum concentration observed | | | | | | | |
| ⁴Time of last nonzero concentration observed | | | | | | | |
| ⁵No drug detected | | | | | | | |

### Example 10 - Composition of an Injection Concentrate

In all of the following examples paclitaxel is used as the taxane component. It should be noted that paclitaxel can be substituted with other taxanes.

### Mixing Instructions for Examples 10.1 through 10.4:

The citric acid is dissolved in the ethanol. The desired amount of Vitamin E TPGS is warmed to approximately 40 °C with stirring. To the warmed Vitamin E TPGS, is added the solvent, ethanol containing citric acid, with stirring while maintaining a temperature of 40 °C. The solution is stirred until uniform and taxanes are added slowly with continued stirring. Upon complete dissolution of the taxanes, the solution is allowed to cool to room temperature. The solution remains fluid even after equilibrating to ambient room temperature.

### Example 10.1:

| COMPONENT | QUANTITY |
|---|---|
| Ethanol | 400 mg |
| Citric Acid | 2mg |
| Vitamin E TPGS | 600 mg |
| Paclitaxel | † |

| | |
|---|---|
| † 6, 10, 20, or 50 mg | |

### Example 10.2:

| COMPONENT | QUANTITY |
|---|---|
| Ethanol | 500 mg |
| Citric Acid | 2 mg |
| Vitamin E TPGS | 500 mg |
| Paclitaxel | † |

| | |
|---|---|
| † 6, 10, 20, or 50 mg | |

### Example 10.3:

| COMPONENT | QUANTITY |
|---|---|
| Ethanol | 625 mg |
| Citric Acid | 2 mg |
| Vitamin E TPGS | 375 mg |
| Paclitaxel | † |

| | |
|---|---|
| † 6, 10, 20, or 50 mg | |

### Example 10.4:

| COMPONENT | QUANTITY |
|---|---|
| Ethanol | 725 mg |
| Citric Acid | 2 mg |
| Vitamin E TPGS | 250 mg |
| Paclitaxel | † |

| | |
|---|---|
| † 6, 10, 20, or 50 mg | |

### Mixing Instructions for Examples 10.5 through 10.8:

The desired amount of Vitamin E TPGS is warmed to approximately 40 °C with stirring. To the warmed Vitamin E TPGS, is added the solvent, ethanol, with stirring while maintaining a temperature of 40 °C. The solution is stirred until uniform and taxanes are added slowly with continued stirring. Upon complete dissolution of the taxanes, the solution is allowed to cool to room temperature. The solution remains fluid even after equilibrating to ambient room temperature.

### Example 10.5:

| COMPONENT | QUANTITY |
|---|---|
| Ethanol | 400 mg |
| Vitamin E TPGS | 600 mg |
| Paclitaxel | † |

| | |
|---|---|
| † 6, 10, 20, or 50 mg | |

### Example 10.6:

| COMPONENT | QUANTITY |
|---|---|
| Ethanol | 500 mg |
| Vitamin E TPGS | 500 mg |
| Paclitaxel | † |

| | |
|---|---|
| † 6, 10, 20, or 50 mg | |

### Example 10.7:

| COMPONENT | QUANTITY |
|---|---|
| Ethanol | 625 mg |
| Vitamin E TPGS | 375 mg |
| Paclitaxel | † |

| | |
|---|---|
| † 6, 10, 20, or 50 mg | |

### Example 10.8:

| COMPONENT | QUANTITY |
|---|---|
| Ethanol | 725 mg |
| Vitamin E TPGS | 250 mg |
| Paclitaxel | † |

| | |
|---|---|
| † 6, 10, 20, or 50 mg | |

### Example 11 - Composition of a Gelatin Capsule

### Mixing Instructions for Examples 11.1 through 11.3:

The citric acid is dissolved in the CREMOPHOR® EL cosurfactant. The desired amount of Vitamin E TPGS is warmed to approximately 40 °C with stirring. To the warmed Vitamin E TPGS, is added the CREMOPHOR® EL/citric acid mixture with stirring while maintaining a temperature of 40° C. To the warmed mixture, is added the solvent of choice with stirring while maintaining a temperature of 40° C. The solution is stirred until uniform and taxanes are added slowly with continued stirring. Upon complete dissolution of the taxanes, the solution is allowed to cool to room temperature.

### Example 11.1:

| COMPONENT | QUANTITY |
|---|---|
| CREMOPHOR® EL | 100 mg |
| Citric Acid | 2 mg |
| Dimethylisosorbide | 250 mg |
| Vitamin E TPGS | 550 mg |
| Paclitaxel | 100 mg |

### Example 11.2:

| COMPONENT | QUANTITY |
|---|---|
| CREMOPHOR® EL | 100 mg |
| Citric Acid | 2 mg |
| Dimethylisosorbide | 300 mg |
| Vitamin E TPGS | 500 mg |
| Paclitaxel | 100 mg |

### Example 11.3:

| COMPONENT | QUANTITY |
|---|---|
| CREMOPHOR® EL | 100 mg |
| Citric Acid | 2 mg |
| Dimethylisosorbide | 250 mg |
| Vitamin E TPGS | 590 mg |
| Paclitaxel | 60 mg |

### Mixing Instructions for Examples 11.4 through 11.8:

The desired amount of Vitamin E TPGS is warmed to approximately 40 °C with stirring. To the warmed Vitamin E TPGS, is added the solvent of choice with stirring while maintaining a temperature of 40 °C. If present, a thickener (e.g. PEG 4600) is added with stirring while maintaining a temperature of 40 °C. The solution is stirred until uniform and taxanes are added slowly with continued stirring. Upon complete dissolution of the taxanes, the solution is allowed to cool to room temperature.

### Example 11.4:

| COMPONENT | QUANTITY |
|---|---|
| Dimethylisosorbide | 200 mg |
| Vitamin E TPGS | 700 mg |
| Paclitaxel | 100 mg |

### Example 11.5:

| COMPONENT | QUANTITY |
|---|---|
| Dimethylisosorbide | 250 mg |
| Vitamin E TPGS | 650 mg |
| Paclitaxel | 100 mg |

### Example 11.6:

| COMPONENT | QUANTITY |
|---|---|
| Methoxy PEG 350 | 250 mg |
| Vitamin E TPGS | 650 mg |
| Paclitaxel | 100 mg |

### Example 11.7:

| COMPONENT | QUANTITY |
|---|---|
| PEG 300 | 250 mg |
| Vitamin E TPGS | 650 mg |
| Paclitaxel | 100 mg |

### Example 11.8:

| COMPONENT | QUANTITY |
|---|---|
| PEG 4600 Flake | 50 mg |
| Dimethylisosorbide | 250 mg |
| Vitamin E TPGS | 700 mg |
| Paclitaxel | 100 mg |

### References

(1989) *The Merck Index* monograph 9040.
(1991) *US. National Cancer Institute's Clinical Brochure for Taxol.*
(1992) Second National Cancer Institute Workshop on Taxol and Taxus held in Alexandria, Virginia USA.
U.S. Patent No. 4,942,184, Issued July 17, 1990.
U.S. Patent No. 4,960,790, Issued October 2, 1990.
U.S. Patent No. 5,733,888, Issued March 31, 1998.

## Claims

1. A composition comprising a taxane and Vitamin E-TPGS and at least one of:
(a) PEG 300, methoxy PEG 350 or PEG 4600;
(b) Dimethylisosorbide (DMI); and
(c) an organic solvent from 40% to 75% of the composition:
Or
A composition comprising 25 to 75% Vitamin E-TPGS, 25 to 75% ethanol, and 0.6 to 5% taxane.

2. The composition of claim 1 comprising at least one of PEG 300, methoxy PEG 350, PEG 4600 or dimethylsorbide.

3. The composition of claims 1 or 2 which further comprises citric acid.

4. The composition of claim 1 wherein the organic solvent is ethanol.

5. The composition of claim 1, comprising 65 to 70% Vitamin E-TPGS, 20 to 25% DMI, and 5 to 10% taxane.

6. The composition of claim 1, comprising 25 to 75% Vitamin E-TPGS, 25 to 75% ethanol, and 0.6 to 5% taxane.

7. The composition of claim 6, further comprising about 0.2% citric acid.

8. The composition of claim 7, further comprising about 10% polyethoxylated castor oil.

9. The composition of claim 1, comprising polyethoxylated castor oil, citric acid, dimethylisosorbide, Vitamin E-TPGS and taxane.

10. The composition of claim 9, comprising about 10% polyethoxylated castor oil, 25 to 30% dimethylisosorbide, 50 to 60% Vitamin E-TPGS about 0.2% citric acid, and 5 to 10% taxane.

11. The composition of claim 1, comprising about 25% methoxy PEG 350, about 65% Vitamin E-TPGS. and about 10% taxane.

12. The composition of claim 1, comprising about 25% PEG 300, about 65% Vitamin E-TPGS, and about 10% taxane.

13. The composition of claim 1, comprising PEG 4600, dimethylisosorbide, Vitamin E-TPGS and taxane.

14. The composition of claim 13, comprising about 5% PEG 4600, about 25% dimethylisosorbide, about 70% Vitamin E-TPGS, and about 10% taxane.

15. The composition of any preceding claim, wherein the taxane is paclitaxel.

16. Use of a taxane and Vitamin E-TPGS for the manufacture of a medicament for treating a taxane-responsive disease condition which medicament comprises a taxane and Vitamin E-TPGS and at least one of:
(a) PEG 300. methoxy PEG 350 or PEG 4600:
(b) Dimethylisosorbide (DMI); and
(c) an organic solvent which if present is from 40% to 75% of the composition;
Or
wherein said medicament comprises 25 to 75% Vitamin E-TPGS, 25 to 75% ethanol, and 0.6 to 5% taxane.

17. The use of claim 16, wherein the medicament is for intravenous or parenteral administration.

18. The use of claim 16 or claim 17, wherein the medicament is the composition of any of claims 2 to 15.

19. The use of any of claims 16 to 18, wherein the taxane-responsive disease condition is selected from ovarian cancer, prostate cancer, breast cancer, malignant lymphoma, lung cancer, melanoma, Kaposi's sarcoma, polycystic kidney disease, Alzheimer's disease, malaria, and rheumatoid arthritis.

## Patentansprüche

1. Zusammensetzung, umfassend ein Taxan und Vitamin E TPGS und mindestens eine Komponente aus:
(a) PEG-300, Methoxy PEG-350 oder PEG-4600,
(b) Dimethylisosorbid (DMI), und
(c) einem organischen Lösungsmittel von 40% bis 75% der Zusammensetzung,
oder
Zusammensetzung, umfassend 25 bis 75% Vitamin E TPGS, 25 bis 75% Ethanol, und 0,6 bis 5% Taxan.

2. Zusammensetzung nach Anspruch 1, umfassend mindestens eine Komponente aus PEG-300, Methoxy PEG-350, PEG-4600 oder Dimethylsorbid.

3. Zusammensetzung nach einem der Ansprüche 1 oder 2, weiterhin umfassend Zitronensäure.

4. Zusammensetzung nach Anspruch 1, wobei das organische Lösungsmittel Ethanol ist.

5. Zusammensetzung nach Anspruch 1, umfassend 65 bis 70% Vitamin E TPGS, 20 bis 25% DMI, und 5 bis 10% Taxan.

6. Zusammensetzung nach Anspruch 1, umfassend 25 bis 75% Vitamin E TPGS, 25 bis 75% Ethanol, und 0,6 bis 5% Taxan.

7. Zusammensetzung nach Anspruch 6, weiterhin umfassend etwa 0,2% Zitronensäure.

8. Zusammensetzung nach Anspruch 7, weiterhin umfassend etwa 10% polyethoxyliertes Castoröl.

9. Zusammensetzung nach Anspruch 1, umfassend polyethoxyliertes Castoröl, Zitronensäure, Dimethylisosorbid, Vitamin E TPGS und Taxan.

10. Zusammensetzung nach Anspruch 9, umfassend etwa 10% polyethoxyliertes Castoröl, 25 bis 30% Dimethylisosorbid, 50 bis 60% Vitamin E TPGS, etwa 0,2% Zitronensäure und 5 bis 10% Taxan.

11. Zusammensetzung nach Anspruch 1, umfassend etwa 25% Methoxy PEG-350, etwa 65% Vitamin E TPGS und etwa 10% Taxan.

12. Zusammensetzung nach Anspruch 1, umfassend etwa 25% PEG-300, etwa 65% Vitamin E TPGS und etwa 10% Taxan.

13. Zusammensetzung nach Anspruch 1, umfassend PEG-4600, Dimethylisosorbid, Vitamin E TPGS und Taxan.

14. Zusammensetzung nach Anspruch 13, umfassend etwa 5% PEG-4600, etwa 25% Dimethylisosorbid, etwa 70% Vitamin E TPGS und etwa 10% Taxan.

15. Zusammensetzung nach einem der vorhergehenden Ansprüche, wobei das Taxan Paclitaxel ist.

16. Verwendung eines Taxans und von Vitamin E-TPGS zur Herstellung eines Arzneimittels für die Behandlung eines auf Taxan ansprechenden Krankheitszustands, wobei das Arzneimittel ein Taxan und Vitamin E TPGS umfasst und mindestens eine Komponente aus:
(a) PEG-300, Methoxy PEG-350 oder PEG-4600,
(b) Dimethylisosorbid (DMI), und
(c) einem organischen Lösungsmittel, welches von 40% bis 75% der Zusammensetzung vorhanden ist,
oder
wobei das Arzneimittel 25 bis 75% Vitamin E TPGS, 25 bis 75% Ethanol, und 0,6 bis 5% Taxan umfasst.

17. Verwendung nach Anspruch 16, wobei das Arzneimittel für intravenöse oder parenterale Verabreichung ist.

18. Verwendung nach Anspruch 16 oder Anspruch 17, wobei das Arzneimittel die Zusammensetzung nach einem der Ansprüche 2 bis 15 ist.

19. Verwendung nach einem der Ansprüche 16 bis 18, wobei der auf Taxan ansprechende Krankheitszustand ausgewählt wird aus Ovarialkrebs, Prostatakrebs, Brustkrebs, malignem Lymphom, Lungenkrebs, Melanom, Kaposi-Sarkom, polycystischer Nierenerkrankung, Morbus Alzheimer, Malaria und Polyarthritis.

## Revendications

1. Composition comprenant un taxane et la vitamine E-TPGS et au moins un composé choisi parmi :
(a) le PEG 300, le méthoxy PEG 350 ou le PEG 4600 ;
(b) le diméthylisosorbide (DMI) ; et
(c) un solvant organique comprenant 40 % à 75 % de la composition :
ou
une composition comprenant 25 à 75 % de vitamine E-TPGS, 25 à 75 % d'éthanol, et 0,6 à 5 % de taxane.

2. Composition selon la revendication 1, comprenant au moins un composé choisi parmi le PEG 300, le méthoxy PEG 350, le PEG 4600 ou le diméthylisosorbide.

3. Composition selon la revendication 1 ou 2 qui comprend en outre de l'acide citrique.

4. Composition selon la revendication 1 dans laquelle le solvant organique est l'éthanol.

5. Composition selon la revendication 1, comprenant 65 à 70 % de vitamine E-TPGS, 20 à 25% de DMI, et 5 à 10 % de taxane.

6. Composition selon la revendication 1, comprenant 25 à 75 % de vitamine E-TPGS, 25 à 75 % d'éthanol, et 0,6 à 5 % de taxane.

7. Composition selon la revendication 6, comprenant en outre 0,2 % d'acide citrique.

8. Composition selon la revendication 7, comprenant en outre environ 10 % d'huile de ricin polyéthoxylée.

9. Composition selon la revendication 1, comprenant de l'huile ricin polyéthoxylée, de l'acide citrique, du diméthylisosorbide, de la vitamine E-TPGS et du taxane.

10. Composition selon la revendication 9, comprenant environ 10 % d'huile de ricin polyéthoxylée, 25 à 30 % de diméthylisosorbide, de 50 à 60 % de vitamine E-TPGS, environ 0,2 % d'acide citrique, et 5 à 10 % de taxane.

11. Composition selon la revendication 1, comprenant environ 25 % de méthoxy PGE 350, environ 65 % de vitamine E-TPGS, et environ 10 % de taxane.

12. Composition selon la revendication 1, comprenant environ 25 % de PEG 300, environ 65 % de vitamine E-TPGS, et environ 10 % de taxane.

13. Composition selon la revendication 1, comprenant du PEG 4600, du diméthylisosorbide, de la vitamine E-TPGS et du taxane.

14. Composition selon la revendication 13, comprenant environ 5 % de PEG 4600, environ 25 % de diméthylisosorbide, environ 70 % de vitamine E-TPGS, et environ 10 % de taxane.

15. Composition selon l'une quelconque des revendications précédentes, dans lequel le taxane est le paclitaxel.

16. Utilisation d'un taxane et de la vitamine E-TPGS pour la fabrication d'un médicament destiné à traiter une maladie sensible au taxane, lequel médicament comprend un taxane et de la vitamine E-TPGS et au moins un composé choisi parmi :
(a) le PEG 300, le méthoxy PEG 350 ou le PEG 4600 ;
(b) le diméthylisosorbide (DMI) ; et
(c) un solvant organique qui, s'il est présent, comprend 40 % à 75 % de la composition :
ou
dans laquelle ledit médicament comprend 25 à 75 % de vitamine E-TPGS, 25 à 75 % d'éthanol, et 0,6 à 5 % de taxane.

17. Utilisation selon la revendication 16, dans laquelle le médicament est destiné à être administré par voie intraveineuse ou parentérale.

18. Utilisation selon la revendication 16 ou la revendication 17, dans laquelle le médicament est la composition selon l'une quelconque des revendications 2 à 15.

19. Utilisation selon les revendications 16 à 18, dans laquelle la maladie sensible au taxane est choisie parmi le cancer ovarien, le cancer de la prostate, le cancer du sein, le lymphome malin, le cancer du poumon, le mélanome, le sarcome de Kaposi, la maladie kystique des reins, la maladie d'Alzheimer, le paludisme, et la polyarthrite rhumatoïde.
